# EUROPEAN PATENT APPLICATION

(11) **EP 1 576 894 A1**
(43) Date of publication of application: **21.09.2005**
(21) Application number: 04075848.4
(22) Date of filing: 16.03.2004
(51) Int. Cl.: A23L 1/30, A61K 31/688, A61K 31/164, A61K 31/133, A61P 3/10

(54) **The use of sphingolipids in the treatment and prevention of type 2 diabetes mellitus, insulin resistance and Metabolic Syndrome**

(71) Applicant: Nederlandse Organisatie voor Toegepast-Natuurwetenschappelijk Onderzoek TNO, 2628 VK Delft (NL)
(72) Inventor: Nieuwenhuizen, Willem Ferdinand, 3981 AM Bunnik (NL); Havekes, Aloysius Maria, 2402 PP Alphen aan den Rijn (NL); Emeis, Josephus Jan, 1018 BE Amsterdam (NL)
(74) Representative: Winckels, Johannes Hubertus F.

(57) **Abstract**

The present invention relates to the use of sphingolipids for the preparation of a food item, a food supplement and/or a medicament for the treatment and/or prevention of insulin resistance, diabetes mellitus type 2 and/or Metabolic Syndrome. In particular, the invention relates to the use of a sphingolipid with the general formula (I): 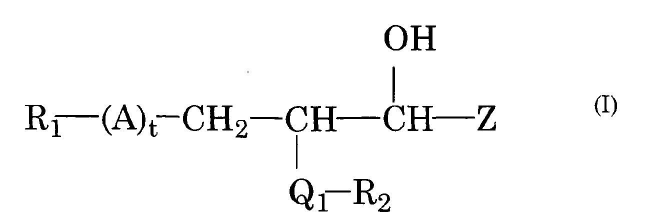 wherein
Z is R₃ or -CH(OH)-R₃;
A is sulphate, sulphonate, phosphate, phosphonate or -C(O)O-;
R₁ is H, hydroxyl, alditol, aldose, an alcohol, C₁-C₆ alkyl or amino acid;
R₂ is H or unsaturated or saturated (C₁-C₃₀) alkyl chain;
R₃ is unsaturated or saturated (C₁-C₃₀) alkyl chain;
Q₁ is a primary amine group (-NH₂), secondary amine group (-NH-) or an amide group (-NH-CO-); and
t is 0 or 1, or a precursor, a derivative or a pharmaceutically acceptable salt thereof, for the manufacture of a medicament for the prevention and/or treatment of a disorder selected from the group consisting of insulin resistance, diabetes type 2 and Metabolic Syndrome.

## Description

### TECHNICAL FIELD

The invention relates to preparations for the treatment and prevention of type 2 diabetes mellitus. In particular, the present invention relates to a food item or food supplement comprising a sphingolipid, to a food item containing this food supplement, to a pharmaceutical preparation comprising a sphingolipid, and to methods for the preparation of the above. The invention further relates to the use of sphingolipids, more preferably phytosphingosine, sphingosine, sphinganine, ceramide, cerebroside and/or sphingomyelin for the preparation of a medicament for the treatment and/or prevention of type 2 diabetes mellitus.

### BACKGROUND OF THE INVENTION

Type 2 diabetes mellitus, formerly called adult-onset or noninsulin-dependent diabetes, is a chronic disease marked by perturbations in both glucose and lipid metabolism. It is widely accepted that insulin resistance and impaired insulin production by pancreatic β-cells are the underlying causes of these perturbations (Kadowaki, 2000). The peptide hormone insulin stimulates the uptake and storage of glucose in skeletal muscle and adipose tissue and it stimulates the synthesis of glycogen (from glucose) and of triglycerides. Simultaneously, insulin inhibits the glucose production in the liver by blocking the gluconeogenesis and glycogenolysis. Defective insulin secretion or insulin resistance will result in malfunctioning of major metabolic pathways and is an important risk factor for acquiring type 2 diabetes.

The condition in which insulin is unable of eliciting its normal anabolic responses at maximal dosage of the hormone is termed insulin resistance (Saltiel, 2001). An inadequate response to insulin leads to decreased glucose uptake (predominantly in muscle) and increased hepatic gluconeogenesis, both of which will cause circulating blood glucose concentrations to rise. To maintain homeostasis and prevent hyperglycemia (excessive serum glucose levels) pancreatic β-cells increase their insulin secretion, causing hyperinsulinemia (high blood insulin levels). Early in the progression to diabetes but before the development of type 2 diabetes, the pancreas is able to overcome insulin resistance and maintain euglycemia by increasing production. Later in the progression to diabetes, the compensatory insulin secretion by the pancreas fails to overcome the insulin resistance of the body and normal plasma glucose concentrations can no longer be maintained, thus resulting in hyperglycemia (Olefsky, 2000; Mayerson & Inzucchi, 2002). The symptoms of hyperglycemia are polyurea (passage of a large volume of urine in a given period) and polydipsia (excessive thirst). Chronic hyperglycemia exerts deleterious effects on pancreatic β-cell-function by means of glucose desensitisation (further reduction of insulin sensitivity of the body, i.e. increased insulin resistance) and exhaustion and apoptosis of β-cells, which impairs insulin secretion (Poitout & Robertson, 2002). This will ultimately result in overt type 2 diabetes, characterized by a fasting venous whole blood glucose concentration of over 7.0 mmol/l (126 mg/dL).

Type 2 diabetes coincides with a marked decrease in life expectancy and is a disproportionately expensive disease that requires long-term medical attention in order to limit the development of short- and long-term complications associated with the disease. These complications include hyperinsulinemia, hyperglycemia, hypoglycemia (serum glucose < 50 mg/dL), ketoacidosis, increased risk of infections, microvascular complications (i.e., retinopathy, nephropathy), neuropathic complications, and macrovascular disease such as cardiovascular disease (CVD) due to severe arteriosclerosis. The morbidity and mortality associated with diabetes is primarily caused by these complications. For instance, diabetes is the major cause of blindness, as well as an important cause of lower-limb amputation and renal disease.

Many patients with type 2 diabetes are asymptomatic and go undiagnosed for many years. Studies suggest that patients with new-onset type 2 diabetes have actually had diabetes for at least 4-7 years before diagnosis. Although type 2 diabetes is found most commonly in adults above the age of 40 with a family history of diabetes, the incidence of disease is increasing more rapidly in adolescents and young adults than in other age groups. It is now estimated that by the year 2010 approximately 250 million people will be affected by type 2 diabetes worldwide (Shulman, 2000). At present, type 2 diabetes is encountered with increasing frequency in younger people, especially in association with obesity. In fact, type 2 diabetes mellitus and obesity are considered to be closely related.

Abnormalities in glucose and lipid (blood fats) metabolism, abdominal obesity, high blood pressure and CVD occur together commonly enough in the same individuals as to suggest that they are somehow interrelated. In fact, this cluster of abnormalities has come to be known as the metabolic syndrome (Hansen, 1999). What seems to connect the various features of the syndrome together is the underlying insulin resistance. In the majority of cases, type 2 diabetes is believed to be a progressive manifestation of the metabolic syndrome (Tenenbaum et *al*., 2003). The other main characteristic factor of the metabolic syndrome apart from the ones mentioned is atherogenic dyslipidemia and the key phase in the manifestation of the disorder is believed to be the insulin resistance. It is held that when the progression of the disease is such that insulin hypersecretion can no longer compensate for insulin resistance and insulin secretion progressively decreases due to stress and damage to pancreatic β-cells, the resulting hyperglycemia is the factual manifestation of type 2 diabetes. Thus, the decrease in insulin secretion that leads to hyperglycemia occurs as a late phenomenon in metabolic syndrome and, in fact, separates the patients with metabolic syndrome from those with diabetes.

This alleged relationship between the metabolic syndrome and type 2 diabetes is supported by the manifestation of mutual risk factors of abdominal obesity and the occurrence of atherogenic dyslipidemia. Atherogenic dyslipidemia (also known as the atherogenic lipoprotein phenotype or lipid triad) is a disorder of lipoprotein metabolism, including lipoprotein overproduction or defective plasma clearance of lipoproteins. It is characterized by elevated serum triglyceride (TG) levels, elevated serum total cholesterol levels, and elevated low-density lipoprotein (LDL) particles, with a concomitant decrease in the high-density lipoprotein (HDL) cholesterol concentration. Dyslipidemia is an integral component of the metabolic perturbations that characterise type 2 diabetes, obesity and the metabolic syndrome, and is intimately associated with premature atherosclerosis and elevated cardiovascular risk. The causal relationship between elevated serum cholesterol levels and the genesis of coronary heart disease is well established. Likewise it is well known that atherogenic dyslipidemia results in increased risk of acquiring cardiovascular disease. The relationship between obesity, insulin resistance and the development of type 2 diabetes on the one hand, and hyperlipidemia and increased cardiovascular risk on the other is becoming ever more clear.

What little is understood of the aetiology of type 2 diabetes, it is clear that it is a multifactorial disease, involving both genetic and environmental factors. In particular, obesity in combination with an unhealthy, fat-rich diet is an important risk factor. Most patients (90%) who develop type 2 diabetes are obese. Numerous studies suggest that the oversupply of lipid to peripheral tissues might contribute to the development of insulin resistance, which allows for the conclusion that excessive energy intake with concomitant obesity is an important risk factor for developing type 2 diabetes (Lewis et *al*., 2002). Obesity is characterized by an excessive amount of adipose tissue. The excess amount of adipose tissue in obese individuals disturbs lipid metabolism, prolonged disturbance leading to dyslipidemia. Because a higher pool of free fatty acids (FFAs) in adipocytes will result in a higher release of FFAs from adipocytes into the circulation, the greater overall fat mass in obese individuals will result in an elevation of the fatty acid flux to non-adipose tissue.

The FFAs released from adipose tissue primarily end up in the liver. There, FFAs are used in β-oxidation, are used in the formation of triglycerides (TG) for fat storage, and are secreted into the bloodstream as very low density lipoproteins (VLDL). An increase in the FFA-flux results in TG-accumulation in the liver and in an increased VLDL-secretion into the bloodstream. The TG-rich VLDL particles deliver the FFAs to other tissues, like skeletal muscle, where it is used as energy by β-oxidation. When the influx of fatty acids in the muscle is higher than the β-oxidation, excessive TG-storage in various parts of the body will result (Lewis et *al*., 2002). Excessive accumulation of TG in the liver is associated with insulin resistance with regards to insulin's normal blocking of hepatic gluconeogenesis and glycogenolysis. In muscle, TG accumulation is also associated with insulin resistance, characterized by a decrease in insulin stimulated glucose uptake (Pan et *al*., 1997). FFA are elevated in many insulin resistant states and have been suggested to contribute to insulin resistance by inhibiting glucose uptake, glycogen synthesis and glucose oxidation and by increasing glucose output. In this way, high serum FAA levels may ultimately contribute to development of insulin resistance. Elevations in free fatty acid (FFA) may thus be an important mechanism underlying the development of diabetes type 2.

It is presently unknown which compounds can effectively be used in the treatment of insulin resistance and, eventually diabetes type 2. Currently, in treatment of type 2 diabetes patients, the clinical manifestation of the diabetes is treated, but not the mechanisms underlying insulin resistance itself. It is found that patients with type 2 diabetes often do not need treatment with oral antidiabetic medication or insulin if they lose weight by successfully adhering to a physician-directed weight loss program including strict diet control and exercise. Dietary measures as well as a clear decrease in body weight are in fact more important than current pharmaceutical options, because an optimal treatment of this metabolic disease can be attained. The initial treatment for these patients is a trial of medical nutrition therapy (MNT; commonly referred to as diet therapy). Appropriate nutritional treatment for insulin resistance is controversial. Two main approaches are drawn from diabetes recommendations: i) a high-carbohydrate, low-fat, high-fibre diet emphasizing low glycemic-index foods and ii) sharing calories between monounsaturated fat and complex carbohydrate at the expense of saturated fat. Promising data have emerged from the first approach, showing that a high-carbohydrate, low-fat, high-fibre diet plus exercise programs maintained through intensive counselling can decrease diabetes risk by over 40% (Sievenpiper et *al*., 2002). At present it is not clear how these remarkable effects of dietary treatment are attained.

In real-life, however, a diet therapy has proven difficult to maintain for patients and they often relapse into their former unhealthy dietary habits. As a result, therapy is in many instances still aimed at the pharmaceutical treatment of the elevated blood sugar and cholesterol values.

Research into the molecular mechanisms of insulin resistance, dyslipidemia, metabolic syndrome and type 2 diabetes has revealed that the mechanism by which insulin resistance is induced may involve alterations in gene expression profiles brought about by transcription factors (Saltiel & Kahn, 2001). A particular group of transcription factors, the so-called peroxisome proliferator activated receptors (PPARs), have recently gained much attention in relation to insulin resistance. Three types of PPARs have been identified: PPARα, PPARβ (PPARδ) and PPARγ PPARα is a member of the steroid hormone receptor super family and is involved in the regulation of lipid metabolism in the liver, heart, kidney and muscles. This makes PPARα a candidate gene for type 2 diabetes and dyslipidemia (Vohl et *al*., 2000). It was suggested that a PPAR-based appraisal of metabolic syndrome and type 2 diabetes may improve the understanding of these diseases and set a basis for a comprehensive approach in their treatment (Tenenbaum *et al.,* 2003). It was further found that dyslipidemia can successfully be treated with fibrates, which are known agonists of PPAR-α (Chapman, 2003). PPAR agonists seem to improve dyslipidemia by regulating the expression of important genes involved in the deranged lipoprotein metabolism associated with insulin resistance (Ruotolo & Howard, 2002). Fibrates effectively lower plasma triglycerides and are widely used in the treatment of hyperlipidemia (Staels et *al*., 1998; Fruchart et *al*., 1998). Although fibrates have been shown to slow the progression of atherosclerosis, and cardiovascular mortality, the results of some trials are ambiguous. Fenofibrate, for instance, a known agonist of PPARα was shown to exhibit antioxidant effect in animal tests. However, the drug had no significant effect on total plasma triglycerides and cholesterol concentrations (Beltowski et *al*., 2002). Moreover, the results of other trials demonstrate increased incidence of arrhythmias, myositis, cerebral hemorhages, deterioration of renal function, cancer, and noncardiovascular mortality in patients receiving these drugs. Therefore, the effect of fibrates on other processes involved in atherogenesis needs to be considered (Beltowski *et al*., 2002) and it is concluded that the prior art is inconclusive about the effect of PPAR agonists on insulin resistance, and that if they are available, they suffer from undesirable side effects.

In studying the development of liver tumors upon exposure to hypolipidemic drugs, plasticizers and herbicides Van Veldhoven and coworkers found that besides linoleic acid, sphingoid bases are possible endogenous ligands of PPAR-α (Van Veldhoven et al., 2000). While sphingenine and sphinganine were identified as strong binding ligands, phosphatidylcholine, sphingomyelin, sphinganine-1-phosphate, ceramide, N-acetyl-sphingenine and N-hexadecanoyl-sphingenine were not able to bind to the receptor. Whether these compounds were agonists or antagonists is not known. In other studies, C₂-ceramide (a short-chain ceramide analog) was found to stimulate lipolysis and to decrease the antilipolytic action of insulin, as a result of which it was believed to be involved in the induction of insulin resistance (Mei *et al*., 2002). Thus, this study suggests that sphingolipids may have a negative effect on the development of insulin resistance. In yet another study on insulin resistance in relation to obesity, it was found that sphingomyelin plays a role in the regulation of PPAR-γ mRNA levels in adipocytes and insulin resistance in subjects correlated with a high sphingomyelin content of the adipocyte plasma membrane (Al-Makdissy et *al.,* 2001). Therefore the prior art is inconclusive about the effect of sphingolipids on insulin resistance.

However, the provision of pharmaceutical compositions or food items which do not merely treat the symptoms of diabetes type 2, but which address the underlying problem of dyslipidemia and insulin resistance are presently highly sought after. The present invention provides a pharmaceutical composition and/or food item which does not merely treat the symptoms of diabetes type 2, but which addresses the underlying problem of dyslipidemia and insulin resistance.

The present inventors have previously found that sphingolipids reduce both cholesterol and triglyceride levels of plasma in ApoE*3Leiden mice. Such mice represent a suitable animal model for studying the effect of drugs and food compounds on plasma cholesterol and triglyceride levels (Volger et *al*., 2001; Post et *al*., 2000). For example, apolipoprotein E*3-Leiden transgenic mice fed with a diet containing up to 1% sphingolipids (specifically phytosphingosine, sphingosine, sphinganine, ceramide, cerebroside and/or sphingomyelin) showed a dramatic reduction (up to 60%) in plasma cholesterol levels and an equally dramatic reduction (up to 50%) in plasma triglyceride levels, compared with control diet. As sphingolipids are natural compounds found in all eukaryotic cells, the inventors previously found that food items and clinically safe medicaments can be prepared based on the sphingolipids, which food items and medicaments have the capacity to reduce TG (triglycerides) and cholesterol levels in a subject with a propensity for or suffering from a lipid-related disorder/disease, and which food items and medicaments do not suffer from undesirable side effects.

### SUMMARY OF THE INVENTION

In relation thereto, the present inventors have now found that food items and clinically safe medicaments comprising sphingolipids may very suitably be used for preventing the development of insulin resistance and/or to alleviate the severity of insulin resistance. Due to this capacity, the food items and medicaments of the present invention may be used in the treatment and prevention of diabetes type 2. Also, the food items and medicaments of the present invention may be used in the treatment and prevention of Metabolic Syndrome.

In one aspect the invention now provides the use of a sphingolipid according to the formula (I) 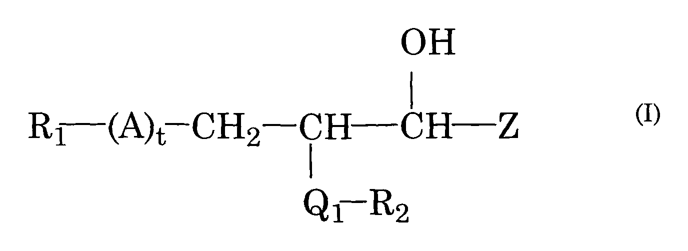 wherein
Z is R₃ or -CH(OH)-R₃;
A is sulphate, sulphonate, phosphate, phosphonate or -C(O)O-;
R₁ is H, hydroxyl, alditol, aldose, an alcohol, C₁-C₆ alkyl or amino acid;
R₂ is H or unsaturated or saturated (C₁-C₃₀) alkyl chain;
R₃ is unsaturated or saturated (C₁-C₃₀) alkyl chain;
Q₁ is a primary amine group (-NH₂), secondary amine group (-NH-) or an amide group (-NH-CO-); preferably an secondary amine group; and t is 0 or 1, or a precursor, a derivative or a pharmaceutically acceptable salt thereof for the manufacture of a medicament for the prevention and/or treatment of a disorder selected from the group consisting of insulin resistance, diabetes type 2 and Metabolic Syndrome.

In a preferred embodiment, said sphingolipid is a sphingolipid according to the formula (II) 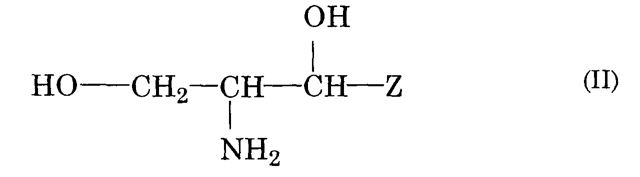 wherein
Z is R₃ or CH(OH)-R₃ and R₃ is an unsaturated or saturated (C₁-C₃₀) alkyl chain, even more preferably a sphingolipid according to formula (III) 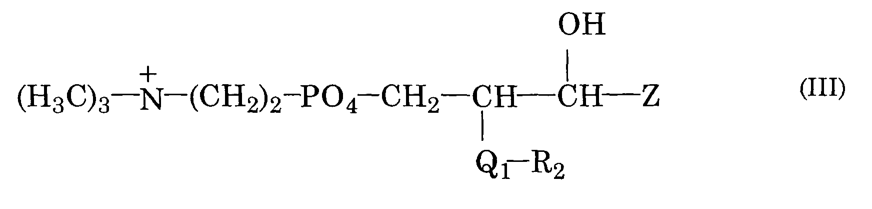 wherein
Z is R₃ or CH(OH)-R₃, preferably R₃, and R₃ is an unsaturated or saturated (C₁-C₃₀) alkyl chain, preferably R₃ is an unsaturated (C₁-C₃₀) alkyl chain;
Q₁ is a primary amine group (-NH₂), a secondary amine group (-NH-) or an amide group (-NH-CO-); preferably an amine group, and
R₂ is H or unsaturated or saturated (C₁-C₃₀) alkyl chain.

In highly preferred embodiments, wherein the sphingolipid is a sphingolipid according to the formula (II), a sphingolipid according to the present invention is phytosphingosine, sphinganine or sphingosine, and in another highly preferred embodiment, wherein the sphingolipid is a sphingolipid according to the formula (III), said sphingolipid is sphingomyelin.

Preferably said disorder is insulin resistance.

The present invention also provides use of a sphingolipid according to the formula (I), (II) or (III) or a precursor or a derivative as an insulin resistance-preventing agent in food items.

In another aspect, the present invention provides a method of preventing the occurrence of insulin resistance, diabetes type 2 and/or Metabolic Syndrome in a healthy subject comprising providing said subject a diet with enhanced levels of a sphingolipid according to the formula (I), (II) or (III) or a precursor, a derivative or a pharmaceutically acceptable salt thereof.

In yet another aspect, the present invention provides a method of treatment of subjects suffering from a disorder selected from the group consisting of insulin resistance, diabetes type 2 and Metabolic Syndrome, said method comprising administrating to subjects in need thereof a therapeutically effective amount of a pharmaceutical composition, said composition comprising a sphingolipid according to the formula (I), (II) or (III), or a precursor, a derivative or a pharmaceutically acceptable salt thereof and a pharmaceutically acceptable carrier, and optionally one or more excipients.

In yet another aspect, the present invention provides the use of a food item with enhanced levels of a sphingolipid according to the formula (I), (II) or (III) or a precursor or a derivative thereof for the prevention and/or treatment of a disorder selected from the group consisting of insulin resistance, diabetes type 2 and Metabolic Syndrome.

In yet another aspect, the present invention provides the use of a food item with enhanced levels of a sphingolipid according to the formula (I), (II) or (III) or a precursor or a derivative thereof in a diet for lowering and/or preventing insulin resistance.

### DEFINITIONS

The term "plasma" as used herein, is the watery, non-cellular portion of the blood from which cellular components, such as red and white blood cells, have been removed usually by centrifugation.

The term "serum" as used herein, is the watery, non-cellular portion of the blood that is left after blood has been clotted and the solids have been removed. Clotting removes blood cells and clotting factors. Serum is thus essentially the same as plasma except that, additionally, clotting factors such as fibrinogen have been removed. Serum and plasma, being watery, contain water-soluble (hydrophilic) substances such as water-soluble vitamins, carbohydrates, and proteins.

As used herein, the term "sphingolipid" includes the generally accepted term of this particular fat-like compound, but it is specifically used to address the group of compounds according to the formulas (I), (II) and (III) of the present invention, including analogs or derivatives or pharmaceutically acceptable salts thereof, alone, or in combination, or as a so-called precursor compound, unless explicitly noted otherwise.

The term "elevated amount" (or "increased amount") relates to an amount of a component in a composition that is higher than the amount of component in the composition in nature or without human intervention. The elevated amount of a component can be caused by addition of a component to a composition which normally does not contain said component, i.e. by enrichment of the composition with said component. An elevated amount of a component can also be caused by addition of a component to a composition which already contains said component, but which has, when the component is added, concentrations of the component which normally do not occur. Also this is called enrichment of the composition with the component.

Because of the variations in the amounts of sphingolipids (such as phytosphingosine, sphingosine, sphinganine, sphingomyelin, ceramide, cerebroside and lyso-sphingomyelin in different food items no general values can be given for the amounts which will be indicated as "elevated amounts" according to the invention. For instance, a small amount of sphingomyelin in potato will be easily called an "elevated amount", because potato from itself does hardly contain any sphingomyelin. The same amount in milk, which normally does contain relatively high concentrations of sphingomyelin, will not give rise to the denomination of "elevated amount".

The term "therapeutically effective amount" as used herein refers to an amount of a therapeutic agent to treat, ameliorate, or prevent a desired disease or condition, or to exhibit a detectable therapeutic or prophylactic effect. The precise effective amount needed for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation.

A "derivative", "analog" or "analogue" is defined herein as a sphingolipid according to the formula (I), (II) or (III) that is subjected to a (bio)chemical modification (e.g. organo-chemical or enzymatical). Derivatising may comprise the substitution of certain chemical groups to the sphingolipid, thereby retaining the sphingolipid character of the compound. Such derivatizations are known in the art. The derivatives and analogues maintain the biological activity of the natural sphingolipid and act in a comparable way, but may provide advantages to the molecule such as longer half-life, resistance to degradation or an increased activity. A very suitable derivative for phytosphingosine is for instance TAPS (see below). Such a derivative may suitably be used in embodiments of the present invention since after hydrolysis, for instance in the body, the converted compound will exert its cholesterol and triglycerides lowering effect.

A "pharmaceutically acceptable salt" is defined herein as a salt wherein the desired biological activity of the sphingolipid is maintained and which exhibits a minimum of undesired toxicological effects. Non-limiting examples of such a salt are (a) acid addition salts formed with inorganic acids (e.g., hydrochloric acid, hydrobromic acid, sulphuric acid, phosphoric acid, nitric acid, and the like), and salts formed with organic acids (such as e.g. acetic acid, oxalic acid, tartaric acid, succinic acid, malic acid, ascorbic acid, benzoic acid, tannic acid, palmitic acid, polyglutamic acid, naphthalene sulphonic acid, naphthalene disulphonic acid, polygalacturonic acid and the like); (b) base addition salts formed with metal cations such as zinc, calcium, bismuth, barium, magnesium, aluminium, copper, cobalt, nickel, cadmium, sodium, potassium and the like, or with a cation formed from ammonia, N,N-dibenzylethylenediamine, D-glucosamine, tetraethylammonium or ethylenediamine; or (c) combinations of (a) and (b); e.g. a zinc tannate or the like. The use of a pharmaceutically acceptable salt of a sphingolipid according to the formula (I), (II) or (III), such as an ammonium salt or a chloride salt is preferred since the salt form is better soluble and will thus enhance the bio-availability of the sphingolipid. Preferably a salt of HCl is used. The use of a pharmaceutically acceptable salt is not limited to pharmaceutical preparations, but includes the use in food items or food supplements.

A "precursor" is defined herein as a derivative of the active compound with similar, less or no activity, and which can be transformed to the active compound e.g. by the digestive tract or other digestive systems in the body. Such precursors can be obtained by chemical or enzymatic modification of the active molecule.

"Subject" as used herein includes, but is not limited to, mammals, including, e.g., a human, non-human primate, mouse, pig, cow, goat, cat, rabbit, rat, guinea pig, hamster, horse, monkey, sheep, or other non-human mammal; and non-mammal animals, including, e.g., a non-mammalian vertebrate, such as a bird (e.g., a chicken or duck) or a fish, and an invertebrate.

### DETAILED DESCRIPTION OF THE INVENTION

Sphingolipids are lipids of which some occur in food in low concentrations and which form a minor but important constituent of the cells of plants, animals and man. Since several sphingolipids occur naturally in the body of man and animal, they will be easily acceptable for addition to food compounds or as pharmaceutical agents.

Sphingolipids are generally composed of a long sphingoid base (sphingosine, sphinganine, phytosphingosine, or a related compound) as the central group of the molecule or "backbone" (see *intra alia* Karlsson. 1970. Chem. Phys. Lipids, 5:6-43), which comprises an amide-linked long-chain fatty acid and a head group. There are hundreds of known classes of sphingolipids with different head groups (e.g. cholinephosphate, glucose, galactose, polysaccharides) and with different fatty acids and sphingoid bases (see *intra alia* Merrill & Sweeley. 1996. New Comprehensive Biochemistry: Biochemistry of Lipids, Lipoproteins, and Membranes, (Vance, D.E. & Vance, J.E., eds.), pp. 309-338, Elsevier Science, Amsterdam).

The simplest sphingolipids, like sphingosine and sphinganine normally occur in food in very low concentrations. The richest sources of sphingolipids are dairy products, eggs, meat and soy beans. The most abundant sphingolipids in our food are sphingomyelin (milk and eggs) and ceramide (meat). Whole milk contains predominantly sphingomyelin, but also contains glucosylceramide, lactosylceramide and gangliosides. Potato, apple, tomato, spinach, pepper and rice especially contain cerebrosides in low concentration (see, e.g. Stryer L., Biochemistry, [W.H. Freeman and Co., NY, USA[, 1988, p. 287 and Ryu J, Kim JS, Kang SS., Cerebrosides from Longan Arillus. Arch Pharm Res. 2003 Feb;26(2):138-42; Kawatake S, Nakamura K, Inagaki M, Higuchi R. Isolation and structure determination of six glucocerebrosides from the starfish *Luidia maculata.* Chem Pharm Bull (Tokyo) 2002 Aug;50(8):1091-6).

It is known that sphingosine and sphingosine-analogs inhibit growth and metastasis of human and animal tumor cells (see e.g. EP 0 381 514). It is also known that administration of sphingomyelin to the food of rats can significantly decrease the chances of occurrence of malignant, chemically induced colon cancer (see Schmeltz, E., *et al.,*).

Sphingolipids are also used in pharmaceutical compositions to protect skin and/or hair against the damaging effects of air pollution (see e.g. US 5.869.034).

The antimicrobial action of sphingosine as a component of the skin against bacteria such as *Staphylococcus aureus, Candida albicans* and *Propionibacterium acnes* is known from dermatology (Bibel et al. 1992. J. Invest. Dermatol. 98(3):269-73; Bibel et al. 1995. Clin Exp Dermatol 20(5):395-400), and the application of topical ointments comprising sphingosine is described therein.

The present inventors have now found that sphingolipids can be used effectively to prevent the development of insulin resistance and/or to alleviate the severity of insulin resistance in a subject when such a sphingolipid is administered to said subject as, for instance, a food item, a food supplement or medicament. Due to there capacity to prevent the development of insulin resistance and/or to alleviate the severity of insulin resistance in a subject, the food items and medicaments of the present invention may also be used in the treatment and prevention of diabetes type 2 and in the treatment and prevention of Metabolic Syndrome.

The present invention now provides in a first aspect the use of a sphingolipid according to the formula (I) 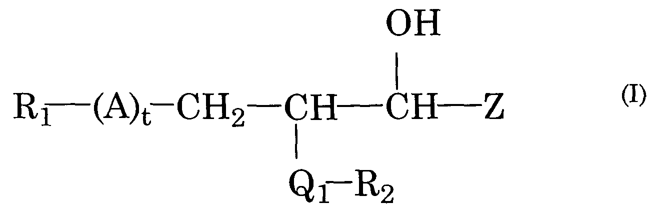 wherein
Z is R₃ or -CH(OH)-R₃;
A is sulphate, sulphonate, phosphate, phosphonate or -C(O)O-;
R₁ is H, hydroxyl, alditol, aldose, an alcohol, C₁-C₆ alkyl or amino acid;
R₂ is H or unsaturated or saturated (C₁-C₃₀) alkyl chain;
R₃ is unsaturated or saturated (C₁-C₃₀) alkyl chain;
Q₁ is a primary amine group (-NH₂), secondary amine group (-NH-) or an amide group (-NH-CO-); preferably an secondary amine group; and t is 0 or 1, or a precursor, a derivative or a pharmaceutically acceptable salt thereof for the manufacture of a medicament for the prevention and/or treatment of a disorder selected from the group consisting of insulin resistance, diabetes type 2 and Metabolic Syndrome.

R₁ can be selected from aldose radicals such as radicals of acesulfam, allose, altrose, arabinose, erythrose, fructose, fucose, galactose, glucose, gulose, idose, isomaltose, lactose, lyxose, maltose, mannose, melezitose, psicose, raffinose, rhamnose, ribose, saccharose, sorbose, stachyose, sucrose, tagatose, talose, threose, trehalose, turanose, xylose and xylulose, and other mono-, di-, or polysaccharides.

R₁ is preferably selected from amino acids radicals, such as radicals of alanine, arginine, asparagines, aspartate, carnitine, citrulline, cysteine, cystine, GABA, glutamate, glutamine, gluthathione, glycine, histidine, hydroxyproline, isoleucine, leucine, lysine, methionine, ornithine, phenylalanine, proline, serine, taurine, threonine, tryptophane, tyrosine and valine or derivatives or combinations thereof.

R₁ is more preferably selected from the group consisting of hydrogen, hydroxyl or hydroxyl-containing group (e.g. hydroxyalkyl), alditol radical or polyol radical, such as radicals of adonitol, arabitol, dulcitol, erythritol, ethyleneglycol, glycerol, inositol, lactitol, maltitol, mannitol, propyleneglycol, ribitol, sorbitol, threitol and xylitol, and of methanol, ethanol, ethanediol, isopropanol, n-propanol, 1,3-propanediol, and other poly-alcohols.

Even more preferably R₁ is selected from the group consisting of radicals of alcohols such as, choline, ethanolamine, ethanol, glycerol, inositol, tyrosine and serine and still more preferably from the alcohol moieties of phosphoglycerides or phosphoglyceride-alcohols, such as choline, serine, ethanolamine, glycerol or inositol.

R₁ is most preferably a hydroxyl group.

(A) can have any desired counter-ion for the formation of a salt of a sphingolipid according to the formula (I).

It is possible that the amino group such as may be present in the form of Q₁ in a sphingolipid according to the formula (I) is modified, e.g. by single or multiple methylation, alkylation, acylation of acetylation or by modification to a formic acid amide.

Also the free hydroxyl groups in the formula (I), specifically those in R₃ may be modified in ways known to the skilled person.

Further, all possible racemates and (dia)stereoisomers of a sphingolipid according to the formula (I) can be used in the present invention. It is possible to use compounds according to the formula (I) wherein Q₁ is substituted by e.g. H, a hydroxyl, a carboxyl or a cyano group. Preferred is a compound wherein Q₁ is the amino group.

R₂ is H or unsaturated or saturated (C₁-C₃₀) alkyl chain and R₃ is unsaturated or saturated (C₁-C₃₀) alkyl chain.

The term alkyl as used herein refers to a saturated or unsaturated straight chain, branched or cyclic, primary, secondary or tertiary hydrocarbon of C₁- C₃₀, optionally substituted, and comprises specifically methyl, ethyl, propyl, butyl, isobutyl, t-butyl, pentyl, cyclopentyl, isopentyl, neopentyl, hexyl, isohexyl, cyclohexyl, cyclohexylmethyl, 3-methylpentyl, 2,2-dimethylbutyl and 2,3-dimethylbutyl, heptyl, octyl, nonyl, decyl, undecyl, dodecyl, tridecyl, tetradecyl, pentadecyl, hexadecyl, heptadecyl, octadecyl, nonadecyl, eikosyl, heneikosyl and dokosyl and isomers thereof.

The C₁- C₃₀ alkyl chain or -group may be optionally substituted with one or more groups selected from the collection consisting of hydroxyl, amino, alkylamino, arylamino, alkoxy, aryloxy, nitro, cyano, sulphonic acid, sulphate, sulphonate, phosphonate or phosphate, either unprotected or protected insofar as desired. These substitutes are known to the person skilled in the art, for example from Greene et al., Protective Groups in Organic Synthesis, John Wiley & Sons, 2^{nd} Edition, 1991. Preferred embodiments of C₁- C₃₀ alkyl chains constitute C₈-C₂₄ alkyl chains.

A compound of the formula (I) is a sphingolipid, or a precursor, a derivative or pharmaceutically acceptable salt thereof.

Even more preferably, in a compound according to the formula (I), or a precursor, a derivative or a pharmaceutically acceptable salt thereof, R₁ is a hydroxyl group, t is 0, R₂ is hydrogen, R₃ is unsaturated or saturated (C₁-C₃₀) alkyl, Q₁-R₂ together is an amine group. More preferably therefore, a sphingolipid used in embodiments of the present invention is a sphingolipid with the general formula (II): 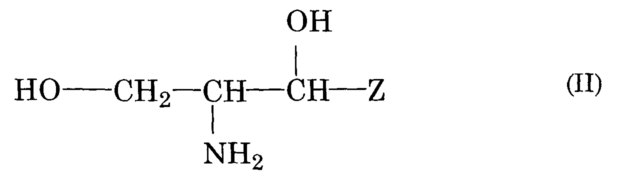 wherein and Z is R₃ or CH(OH)-R₃ and R₃ is an unsaturated or saturated (C₁-C₃₀) alkyl chain.

In a most preferred embodiments of the present invention, a phytosphingosine, sphingosine, sphinganine, ceramide, cerebroside and/or sphingomyelin is used, since these compounds show excellent reduction in plasma cholesterol and triglycerides.

Besides sphingomyelin, phytosphingosine, sphingosine, sphinganine, ceramide and cerebroside also derivatives of these compounds may be used in aspects of the present invention. For instance, in stead of a hydroxyl headgroup, a choline phosphate, ethanolamine phosphate, serine phosphate, inositol phosphate, glycerol phosphate, glucose or galactose head group may be used as R₁ group in a compound according to the formula (I). Basically all headgroups within the definition of R1 above may be used for derivatization of phytosphingosine, sphingosine and sphinganine. A derivative such as lyso-sphingomyelin may also be used in embodiments of the present invention.

It is also possible to use a combination of sphingolipids according to the formula (I) and/or (II) and/or (III) in aspects of the present invention.

In principle, sphingolipids according to the formula (I) and/or (II) and/or (III) of all possible sources are suitable for use in aspects and embodiments of the present invention. For instance, a suitable sphingolipid such as phytosphingosine may be obtained from plants such as corn (Wright et al., Arch. Biochem. Biophys. 415(2), 184-192 and references therein), from animals (skin fibroblasts) or from microorganisms such as yeasts (such as *Pichia ciferii*). The sphingolipids may be isolated from these organisms or can be used in a less pure form, i.e. as an enriched fraction, or in the case of microorganisms such as yeasts by taking the complete organism(s) or fractions thereof. Further, sphingolipids may be isolated from other suitable sources, such as from milk, egg, soy, yeast, bacteria, algae, plants, meat, brain, etc. or may be chemically or enzymatically prepared, for use in a food item, food supplement and/or pharmaceutical composition according to the invention.

For application in a food item or food supplement according to the present invention a sphingolipid is preferably derived from a food-grade source. Examples of suitable food-grade sources are e.g. bakery yeast, brewers yeast and egg, and certain types of bacteria, (filamentous) fungi, sponges and algae, in particular, but not exclusively those species of bacteria, yeast and fungi which are generally recognized as safe (GRAS). Bacterial sources of sphingolipids are e.g. known from US 6,204,006.

Sphingolipids may be derived from the above sources by methods known to the skilled person for instance by extraction with (organic) solvents, chromatographic separation, precipitation, crystallization and/or enzymatic of chemical hydrolysis. The production of a sphingolipid-enriched (specifically a sphingomyelin-enriched) fraction from milk is for instance known from WO94/18289. Sphingolipids may also be derived from fat concentrates of various animal products such as milk products, egg products and blood products such as known from US 5,677,472.

Methods for the preparation of sphingolipids and sphingolipid derivatives are *i*.*a*. known from EP 0 940 409, WO 98/03529, WO 99/50433 and US 6,204,006 and the artisan will be capable of preparing derivatives by these and other methods. Various routes for obtaining sphingosines are described by D. Shapiro in "Chemistry of Sphingolipids", Hermann, Paris (1969). Methods for producing certain phytosphingolipid derivatives are known to the skilled person, for instance it is known from US 6,204,006 and US 5,618,706 to derive tetraacetyl-phytosphingosine (TAPS) from microbial sources (i.e. *Pichia ciferrii)* and to subject this TAPS to hydrolysis to yield phytosphingosine.

A sphingolipid according to the formula (I), or a precursor, a derivative or a pharmaceutically acceptable salt thereof, may also be synthesized by known methods such as e.g. known from U.S. patents 5.232.837 and 5.110.987, or by standard modifications of these methods.

A known issue relating to the administration of sphingolipids, be it in foods or in pharmaceutical compositions, is that they can be metabolized. This is particularly relevant for application of sphingolipids in the digestive tract. This issue may be addressed by administering a sphingolipid according to the formula (I), more preferably according to formula (II) or (III), or a derivative or a pharmaceutically acceptable salt thereof, alone or in combination, as a so-called precursor compound which compound comprises certain substituents as a result of which the compound can no longer, or only at reduced rates, be metabolized. These precursors are preferably resistant to hydrolysis in the upper parts of the digestive tract (e.g. mouth, stomach), and are for instance split relatively easy in the lower part of the digestive tract (e.g. coecum, colon), if the sphingolipid should have its working especially there. Preferably, when the intake of the precursor is via the oral route, the intact or metabolized precursors are taken up into the blood stream and transported to the target organs, especially liver, muscle and adipose tissue where they may be activated in order to exert their beneficial effect. Thus, it is possible that activation occurs when the compound has been absorbed from the digestive tract, e.g. in the serum or the liver. As a result, the amount of the compound is raised at those locations where the sphingolipid has its action. For instance, a sphingolipid precursor may be used that can be split or activated *in vivo* by a suitable enzyme so that the sphingolipid is liberated that may reduce the levels of cholesterol and triglycerides in the subject. Sphingolipid precursors have been described in WO 99/41266.

It is possible to modify a precursor of a sphingolipid according to the formula (I), (II) or (III) by an *in situ* enzymatic or chemical conversion, i.e. in the body, to a sphingolipid according to the formula (I), (II) or (III), which can be used in embodiments of the present invention. Such precursors of a sphingolipid according to the formula (I), (II) or (III) are therefore also suited for use according to the invention. A condition is that the precursor is converted in the body, e.g. preferably in the intestine, to a sphingolipid according to the formula (I), (II) or (III), e.g. by enzymatic conversion, in which case there is *in situ* activation. It is therefore, for instance possible to administer together with e.g. sphingomyelin, the enzyme sphingomyelin deacylase which may convert the sphingomyelin to lyso-sphingomyelin. Another possibility is to use sphingomyelinase to convert sphingomyelin into ceramide. In its turn ceramide can be broken down by ceramidase into a sphingoid base structure and a fatty acid. Other examples of enzymes may for instance be found in Sueyoshi *et al.,* (Sueyoshi et *al*., 1997). Preferably, however, the sphingolipid according to the formula (I), (II) or (III) is not used as a precursor but in its "active" form in a food item or a food supplement or a pharmaceutical preparation.

A sphingolipid according to the formula (I), (II) or (III), or a precursor, a derivative or a pharmaceutically acceptable salt thereof, may be provided to a subject in need thereof for prophylactic or therapeutic reasons. A sphingolipid according to the formula (I), (II) or (III), or a precursor, a derivative or a pharmaceutically acceptable salt thereof, may be provided to a subject in need thereof in the form of a food item or food supplement, or in the form of a pharmaceutical preparation, all such administration forms being capable of preventing the development and/or to alleviate the severity of a disorder selected from the group consisting of insulin resistance, diabetes type 2 and Metabolic Syndrome. In particular, the development and/or severity of insulin resistance is considered.

A sphingolipid according to the formula (I), more preferably according to formula (II), yet more preferably according to formula (III), or a precursor, a derivative or a pharmaceutically acceptable salt thereof, may be used in a food item or food supplement. A food supplement is defined as a composition that can be consumed in addition to the normal food intake and which comprises elements or components that are not or in only minor amounts, present in the normal diet and of which sufficient or increased consumption is desired. The composition of a food item does not necessarily differ much from that of a food supplement.

A food item or food supplement as disclosed herein comprises an amount of sphingolipids according to the formula (I), (II) or (III) that is higher than the amount that would normally or without human intervention occur or be found in said food item or food supplement. This elevated amount of a sphingolipid according to the formula (I), (II) or (III) may arise through specific addition of said sphingolipid to a food item that does not normally comprise said sphingolipid in said elevated amount, i.e. by enrichment of the food item with said sphingolipid. Alternatively genetic engineering may be used to produce food items comprising said sphingolipid in an elevated amount, for instance by engineering the biosynthetic routes for the production of such sphingolipids in a plant, or yeast or other microorganism used for the production of a food item in such a way that said sphingolipid is produced in said organism in an elevated amount.

Since amounts of sphingolipids such as phytosphingosine, sphingosine, (lyso-)sphingomyelin or sphinganine may differ considerably between various food items there is no general value for the amount which is said to be an elevated amount or of an enriched food item. In general, milk, which normally contains relatively high amounts of sphingomyelin, is said to comprise an elevated amount at higher absolute concentrations than for instance a potato, which contains no or only minute amounts of sphingomyelin.

A sphingolipid-enriched food item or food supplement as described above may suitably comprise 0.01 to 99.9 wt.% of a sphingolipid according to the formula (I), (II) or (III). In a preferred embodiment such a food item or food supplement comprises from 0.01 to 50 wt.%, preferably from 0.01 to 10 wt.%, more preferably from 0.01 tot 5 wt.% of a sphingolipid according to the formula (I), (II) or (III) or derivatives, precursors or acceptable salts thereof.

In order to make a food item or food supplement comprising an elevated amount a sphingolipid according to the formula (I), (II) or (III) suitable for human or animal consumption, the nutritional value, texture, taste or smell may be improved by adding various compounds to said item or supplement. The skilled person is well aware of the different sources of protein, carbohydrate and fat that may be used in food items or food supplements according to the invention and of the possible sweeteners, vitamins, minerals, electrolytes, coloring agents, odorants, flavoring agents, spices, fillers, emulsifiers, stabilizers, preservatives, anti-oxidants, food fibers, and other components for food items that may be added to improve its nutritional value, taste or texture. The choice for such components is a matter of formulation, design and preference. The amount of such components and substances that can be added is known to the skilled person, wherein the choice may e.g. be guided by recommended daily allowance dosages (RDA dosages) for children and adults and animals.

Portions for intake of the food item or food supplement may vary in size and are not limited to the values corresponding to the recommended dosages. The term "food supplement" is herein not intended to be limited to a specific weight or dosage.

A composition of a food item or food supplement as described above may in principle take any form suited for consumption by man or animal. In one embodiment the composition is in the form of a dry powder that can be suspended, dispersed, emulsified or dissolved in an aqueous liquid such as water, coffee, tea, milk, yogurt, stock or fruit juice and alcoholic drinks. To this end, the powder may be provided in unit-dosage form.

In an alternative preferred embodiment a composition in the form of a dry powder is tabletted. To that end, a composition for a food supplement according to the invention may very suitably be provided with fillers, such as microcrystalline cellulose (MCC) and mannitol, binders such as hydroxypropylcellulose (HPC), and lubricants such as stearic acid or other excipients.

A composition of a food item or food supplement as described above may also be provided in the form of a liquid preparation wherein the solids are suspended, dispersed or emulsified in an aqueous liquid. Such a composition may be admixed directly through a food item or may e.g. be extruded and processed to grains or other shapes.

In an alternative embodiment a food item or food supplement may take the shape of a solid, semi-solid or liquid food item, such as a bread, a bar, a cookie or a sandwich, or as a spread, sauce, butter, margarine, dairy product, and the like. Preferably, a sphingolipid according to the present invention is applied in a dairy product, such as for instance a butter or margarine, custard, yogurt, cheese, spread, drink, or pudding or other dessert. The sphingolipid can also be used in butters or fats used for frying and baking, because they are relatively stable and will not be degraded by high temperatures. This characteristic also enables use of the sphingolipid in food items or food supplements which undergo a pasteurization or sterilization treatment. Diet products also constitute preferred embodiments of food items or food supplements according to the invention.

If a food item according to the invention is used as an animal feed, the food item may e.g. be prepared in the form of a powder, a grain, a waffle, a porridge, a block, a pulp, a paste, a flake, a cook, a suspension or a syrup.

For administration to humans the food item of the invention may very suitably be prepared in the form of a food supplement.

The present invention further relates to a method for the preparation of a food item or food supplement according to the invention, comprising enriching a food item or food supplement with a sphingolipid according to the formula (I) and/or (II) and/or (III), or a precursor, a derivative or a pharmaceutically acceptable salt thereof.

In one embodiment the invention provides a method for the preparation of a food item or food supplement enriched with a sphingolipid, comprising processing a sphingolipid according to the formula (I), (II) or (III), or a precursor, a derivative or a pharmaceutically acceptable salt thereof in a food item or food supplement, preferably to an amount of 0.01 to 99.9 wt.%, more preferably to an amount of from 0.01 to 50 wt.%, even more preferably to an amount of from 0.01 tot 10 wt.%, and most preferably to an amount of from 0.01 tot 5 wt.%. The amount of sphingolipid processed in a food item according to the invention depends on the type of sphingolipid and its use and the skilled person is capable of determining this amount in the context of the present disclosure.

In a method for preparing a food item according to the invention the food item may first be prepared separately and then be joined with a sphingolipid to provide a food item according to the invention wherein said sphingolipid is incorporated in the food item. The food item may be separately prepared by conventional methods such as by mixing, baking, frying, cooking, steaming or poaching and may, if necessary, be cooled prior to joining with the sphingolipid. According to another suitable embodiment, the sphingolipid is incorporated as a component in the food item during the preparation thereof.

A food item or food supplement according to the present invention may very suitably be defined as a nutraceutical composition. Nutraceuticals can be defined as natural products that are used to supplement the diet by increasing the total dietary intake of important nutrients. This definition includes nutritional supplements such as vitamins, minerals, herbal extracts, antioxidants, amino acids, and protein supplements. Nutraceutical products fit into the newly created product category of "Dietary Supplements" as established by the F.D.A. in the Dietary Supplement Act of 1994. This act specifically defined dietary supplements to include: vitamins, minerals, herbs or other botanicals, antioxidants, amino acids, or other dietary substances used to supplement the diet by increasing the total daily intake.

A "nutraceutical composition" is defined herein as a food composition fortified with ingredients capable of producing health benefits. Such a composition in the context of the present invention may also be indicated as foods for special dietary use; medical foods; and dietary supplements.The food item and/or food supplement of the present invention is a nutraceutical composition since it is fortified with one or more sphingolipids according to the invention and since it is capable of treating or preventing insulin resistance, diabetes type 2 and/or Metabolic Syndrome.

The present invention also relates to a method of treatment of subjects suffering from insulin resistance, diabetes type 2 and/or Metabolic Syndrome said method comprising administering to subjects in need thereof a therapeutically effective amount of a pharmaceutical composition, said composition comprising a sphingolipid according to the formula (I), more preferably according to formula (II), yet more preferably according to the formula (III), most preferably phytosphingosine, sphingosine, sphinganine, cerebrosides, ceramide, or sphingomyelin or precursors, derivatives or pharmaceutically acceptable salts thereof and a pharmaceutically acceptable carrier, and optionally one or more excipients.

The pharmaceutical composition may also comprise a suitable pharmaceutically acceptable carrier and may be in the form of a capsule, tablet, lozenge, dragee, pill, droplet, suppository, powder, spray, vaccine, ointment, paste, cream, inhalant, patch, aerosol, and the like. As pharmaceutically acceptable carrier, any solvent, diluent or other liquid vehicle, dispersion or suspension aid, surface active agent, isotonic agent, thickening or emulsifying agent, preservative, encapsulating agent, solid binder or lubricant can be used which is most suited for a particular dosage form and which is compatible with the sphingolipid.

A pharmaceutical composition may also contain a pharmaceutically acceptable carrier. The term "pharmaceutically acceptable carrier" refers to a carrier for administration of the therapeutic agent. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

For therapeutic treatment, sphingolipid may be produced as described above and applied to the subject in need thereof. The sphingolipid may be administered to a subject by any suitable route, preferably in the form of a pharmaceutical composition adapted to such a route and in a dosage which is effective for the intended treatment. Therapeutically effective dosages of the sphingolipid required for treating the disorder, for instance for prevention and/or treatment of a disorder selected from the group consisting of insulin resistance, diabetes type 2 and Metabolic Syndrome in the body of a human or animal subject, can easily be determined by the skilled person, for instance by using animal models.

The term "therapeutically effective amount" as used herein refers to an amount of a therapeutic, *viz*. a sphingolipid according to the present invention, to reduce or prevent insulin resistance, diabetes type 2 and/or Metabolic Syndrome, or to exhibit a detectable therapeutic or prophylactic effect. The effect can be detected by, for example, measurement of blood sugar, serum triglycerides and/or cholesterol as described herein or by any other suitable method of assessing the progress or severity of insulin resistance, diabetes type 2 and/or Metabolic Syndrome. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician or experimenter. Specifically, the compositions of the present invention can be used to reduce or prevent insulin resistance, diabetes type 2 and/or Metabolic Syndrome and/or accompanying biological or physical manifestations. Methods that permit the clinician to establish initial dosages are known in the art. The dosages determined to be administered must be safe and efficacious.

For purposes of the present invention, an effective dose will be from about 0.01 mg/kg to 1 g/kg, preferably 0.5 mg/kg to about 100 mg/kg, more preferably from about 1 mg/kg to about 25 mg/kg per day of the sphingolipid in the individual to which it is administered.

Yet in another alternative embodiment, the sphingolipid or compositions of the invention may be administered from a controlled or sustained release matrix inserted in the body of the subject.

Dosages for achieving the therapeutic effects of the pharmaceutical composition, food item or food supplement described herein may easily be determined by the skilled person. For purposes of the present invention, an effective dose will be from about 0.01-5% of the dry food weight in the individual to which it is administered, meaning that for an adult human being the daily dose will be between about 0.002 and 10 grams of sphingolipid.

Preferably a pharmaceutical composition as described above is intended for oral application. Compositions for oral application will usually comprise an inert diluent or an edible carrier. The compositions may be packed in e.g. gelatin capsules or may be tabletted in the form of tablets. For oral therapeutic application the active compound may be administered with excipients and e.g. used in the form of powders, sachets, tablets, pills, pastilles or capsules. Pharmaceutically acceptable binders and/or adjuvants may also be comprised as constituents of the pharmaceutical composition.

The powders, sachets, tablets, pills, pastilles, capsules and such may comprise each of the following components or compounds of similar import: a filler such as microcrystalline cellulose (MCC) or mannitol; a binder such as hydroxypropylcellulose (HPC), tragacanth gum or gelatin; an excipient such as starch or lactose; a desintegrant such as alginate or corn starch; a lubricant such as magnesium stearate; a sweetener such as sucrose or saccharose; or a flavoring substance such as peppermint or methyl salicylic acid.

When dosing is in the form of a capsule, the capsule may comprise apart from the elements mentioned above a liquid carrier such as an oil. Dosage form may further be provided with coatings of sugar, shellac or other agents. The components of the pharmaceutical composition are preferably chosen such that they do not reduce the desired working of the sphingolipid.

A sphingolipid according to the formula (I), (II) or (III) or the pharmaceutically acceptable salt thereof may also be administered in the form of e.g. an elixir, a suspension, a syrup, a waffle or a chewing gum.

In a pharmaceutical composition as described above, a sphingolipid according to the formula (I), (II) or (III), or a precursor, a derivative or a pharmaceutically acceptable salt thereof, is used in an amount of from 0.01 to 99.9 % by weight, preferably from 0.01 to 10 wt.%, and more preferably from 0.01 to 1 wt.%.

A pharmaceutical composition according to the invention is intended for treating or preventing insulin resistance in a subject.

The present invention further relates to a method for the preparation of a pharmaceutical composition for the prevention and/or treatment of a disorder selected from the group consisting of insulin resistance, diabetes type 2 and Metabolic Syndrome in a subject, comprising processing or incorporating a sphingolipid according to the formula (I), (II) or (III), or a precursor, a derivative or a pharmaceutically acceptable salt thereof, as an active substance, together with a pharmaceutically acceptable carrier in a pharmaceutical composition.

The preparation of a pharmaceutical composition may very suitably occur by mixing all separate ingredients such as fillers, binders, lubricants and optionally other excipients together with a sphingolipid according to the formula (I), (II) or (III) or a precursor, a derivative or a pharmaceutically acceptable salt thereof, and processing the mixture obtained to a pharmaceutical preparation.

### REFERENCES

**Al-Makdissy N, Bianchi A, Younsi M**, **Picard E, Valet P, Martinet N**, **Dauca M, Donner M.** 2001. Down-regulation of peroxisome proliferator-activated receptor-gamma gene expression by sphingomyelins. *FEBS Lett.* **493**(2-3):75-9.
**Beltowski J, Wojcicka G, Mydlarczyk M, Jamroz A.** 2002. The effect of peroxisome proliferator-activated receptors alpha (PPARalpha) agonist, fenofibrate, on lipid peroxidation, total antioxidant capacity, and plasma paraoxonase 1 (PON 1) activity. *JPhysiol Pharmacol.* **53**(3):463-75.
**Chapman MJ.** 2003. Fibrates in 2003: therapeutic action in atherogenic dyslipidemia and future perspectives. *Atherosclerosis* **171**:1-13.
**Fruchart JC, Brewer HB Jr, Leitersdorf E.** 1998. Consensus for the use of fibrates in the treatment of dyslipoproteinemia and coronary heart disease. Fibrate Consensus Group. *Am J Cardiol.* **81**(7):912-7.
**Hansen BC.** (1999) The metabolic syndrome X. *Ann. N. Y. Acad. Sci.* **892**:1-24
**Kadowaki T.** 2000. Insights into insulin resistance and type 2 diabetes from knockout mouse models. *J. Clin. Invest* **106**(4):459-65.
**Kahn BB, Flier JS.** 2000. Obesity and insulin resistance. *J Clin Invest.* **106**(4):473-81.
**Lewis GF, Carpentier A, Adeli K, Giacca A.** 2002. Disordered fat storage and mobilization in the pathogenesis of insulin resistance and type 2 diabetes. *Endocr Rev.* **23**(2):201-29.
**Mayerson AB, Inzucchi SE.** 2002. Type 2 diabetes therapy. A pathophysiologically based approach, *Postgraduate Medicine* 111(3): 83-95
**Mei J, Stenson Holst L., Rahn Landström T, Holm C, Brindley D, Manganiello V, Degerman E.** 2002. C₂-Ceramide influences the expression and insulin-mediated regulation of cyclic nucleotide phosphodiesterase 3B and lipolysis in 3T3-L1 adipocytes. *Diabetes* **51**: 631-637
**Olefsky JM.** 2000. Treatment of insulin resistance with peroxisome proliferator-activated receptor gamma agonists. *J*. *Clin. Invest* **106**(4):467-72.
**Pan DA, Lillioja S, Kriketos AD, Milner MR, Baur LA, Bogardus C, Jenkins AB, Storlien LH. 1997.** Skeletal muscle triglyceride levels are inversely related to insulin action. *Diabetes.* **46**(6):983-8.
**Poitout V, Robertson RP.** 2002. Minireview: Secondary beta-cell failure in type 2 diabetes--a convergence of glucotoxicity and lipotoxicity. *Endocrinology.* **143**(2):339-42.
**Post SM, De Roos B, Vermeulen M, Afman L, Jong MC, Dahlmans VEH, Havekes LM, Stellaard F, Katan MB, Princen HMG.** Cafestol increases serum cholesterol levels in apolipoprotein E*3-Leiden transgenic mice by suppression of bile acid synthesis. *Arterioscl. Thromb. Vasc. Biol.* **20**:1551-1556
**Ruotolo G, Howard BV.** 2002. Dyslipidemia of the metabolic syndrome. *Curr Cardiol Rep.* 4(6):494-500.
**Saltiel AR.** 2001. New perspectives into the molecular pathogenesis and treatment of type 2 diabetes. *Cell* **104**(4):517-529
**Saltiel AR, Kahn CR.** 2001. Insulin signalling and the regulation of glucose and lipid metabolism. *Nature* **414**(6865):799-806.
**Shulman** GI. 2000. Cellular mechanisms of insulin resistance.*J Clin Invest.* **106**(2):171-6.
**Sievenpiper JL, Jenkins AL, Whitham DL, Vuksan V. 2002.** Insulin resistance: concepts, controversies, and the role of nutrition. *Can J Diet Pract Res.* **63**(1):20-32.
**Staels B, Dallongeville J, Auwerx J, Schoonjans K, Leitersdorf E, Fruchart J-C.** 1998 Mechanism of Action of Fibrates on Lipid and Lipoprotein Metabolism. *Circulation.* **98**:2088-2093.
**Sueyoshi N, Izu H, Ito M.** 1997. Preparation of a naturally occurring D-erythro-(2S, 3R)-sphingosylphosphocholine using Shewanella alga NS-589. J *Lipid Res.* **38**(9):1923-7.
**Tenenbaum A, Fisman EZ, Motro M.** 2003. Metabolic syndrome and type 2 diabetes mellitus: focus on peroxisome proliferator activated receptors (PPAR). *Cardiovasc Diabetol.* **2**(1):4.
**Van Veldhoven PP, Mannaerts GP, Declercq P, Baes M.** 2000. Do sphingoid bases interact with the peroxisome proliferator activated receptor alpha (PPAR-alpha)? *Cell Signal.* **12**(7):475-9.
**Vohl MC, Lepage P, Gaudet D, Brewer CG, Betard C, Perron P, Houde G, Cellier C, Faith JM, Despres JP, Morgan K, Hudson TJ.** 2000. Molecular scanning of the human PPARa gene: association of the L162v mutation with hyperapobetalipoproteinemia. *J Lipid Res.* **41**(6):945-52.
**Volger OL, van der Boom H, de Wit ECM, van Duyvenvoorde W,** Hornstra G, Plat J, Havekes LM, Mensink RP, Princen HMG. 2001. Dietary plant stanol esters reduce VLDL-cholesterol secretion and bile saturation in apoE*3Leiden transgenic mice. *Arterioscler Thromb Vasc Biol.* **21**:1046-1052;

## Claims

1. Use of a sphingolipid with the general formula (I): 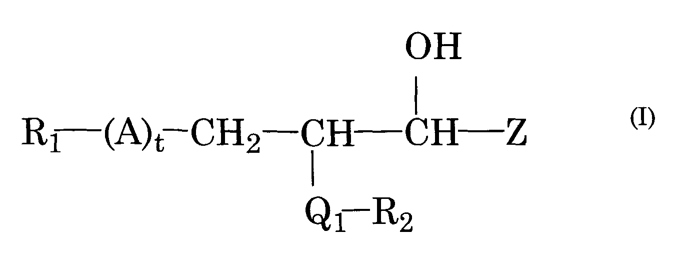 wherein
Z is R₃ or -CH(OH)-R₃;
A is sulphate, sulphonate, phosphate, phosphonate or -C(O)O-;
R₁ is H, hydroxyl, alditol, aldose, an alcohol, C₁-C₆ alkyl or amino acid;
R₂ is H or unsaturated or saturated (C₁-C₃₀) alkyl chain;
R₃ is unsaturated or saturated (C₁-C₃₀) alkyl chain;
Q₁ is a primary amine group (-NH₂), secondary amine group (-NH-) or an amide group (-NH-CO-); and
t is 0 or 1, or a precursor, a derivative or a pharmaceutically acceptable salt thereof, for the manufacture of a medicament for the prevention and/or treatment of a disorder selected from the group consisting of insulin resistance, diabetes type 2 and Metabolic Syndrome.

2. Use of sphingolipid with the general formula (II) 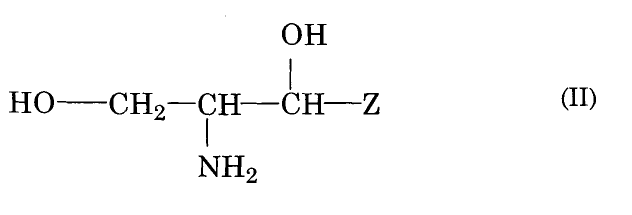 wherein
Z is R₃ or CH(OH)-R₃, and
R₃ is an unsaturated or saturated (C₁-C₃₀) alkyl chain, or a precursor, a derivative or a pharmaceutically acceptable salt thereof,
for the manufacture of a medicament for the prevention and/or treatment of a disorder selected from the group consisting of insulin resistance, diabetes type 2 and Metabolic Syndrome.

3. Use of sphingolipid with the general formula (III) 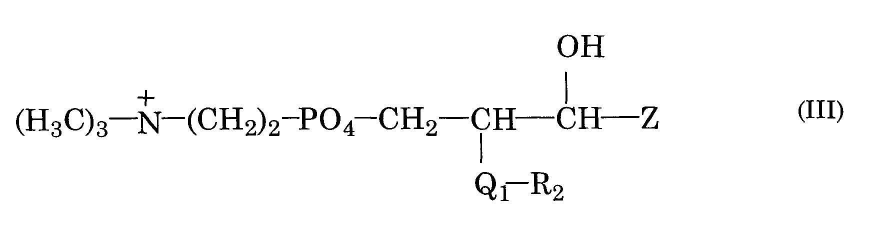 wherein
Z is R₃ or CH(OH)-R₃, preferably R₃;
Q₁ is a primary amine group (-NH₂), a secondary amine group (-NH-) or an amide group (-NH-CO-); preferably an amide group, and
R₂ is H or unsaturated or saturated (C₁-C₃₀) alkyl chain;
R₃ is an unsaturated or saturated (C₁-C₃₀) alkyl chain, preferably an unsaturated (C₁-C₃₀) alkyl chain,
or a precursor, a derivative or a pharmaceutically acceptable salt thereof,
for the manufacture of a medicament for the prevention and/or treatment of a disorder selected from the group consisting of insulin resistance, diabetes type 2 and Metabolic Syndrome.

4. Use of a sphingolipid according to the formula (I) as defined in claim 1 or formula (II) as defined in claim 2, or formula (III) as defined in claim 3, or a precursor or a derivative thereof as a insulin resistance preventing agent in food items.

5. Use according to claim 2, wherein said sphingolipid is phytosphingosine, sphingosine, sphinganine, ceramide, cerebroside and/or sphingomyelin.

6. Use according to claim 3, wherein said sphingolipid is sphingomyelin.

7. Method of preventing the occurrence of insulin resistance, diabetes type 2 and/or Metabolic Syndrome in a healthy subject comprising providing said subject a diet with enhanced levels of a sphingolipid as defined in any one of claims 1-6 or a precursor, a derivative or a pharmaceutically acceptable salt thereof.

8. Method of treatment of a subject suffering from insulin resistance, diabetes type 2 and/or Metabolic Syndrome, said method comprising administrating to a subject in need thereof a therapeutically effective amount of a pharmaceutical composition, said composition comprising a sphingolipid according to the formula (I) as defined in claim 1, or formula (II) as defined in claim 2, or formula (III) as defined in claim 3, or a precursor, a derivative or a pharmaceutically acceptable salt thereof and a pharmaceutically acceptable carrier, and optionally one or more excipients.

9. Use of a food item with enhanced levels of a sphingolipid according to the formula (I) as defined in claim 1, or formula (II) as defined in claim 2, or formula (III) as defined in claim 3, or a precursor or a derivative thereof for the prevention and/or treatment of a disorder selected from the group consisting of insulin resistance, diabetes type 2 and Metabolic Syndrome.

10. Use of a food item with enhanced levels of a sphingolipid according to the formula (I) as defined in claim 1, or formula (II) as defined in claim 2, or formula (III) as defined in claim 3, or a precursor or a derivative thereof in a diet for lowering and/or preventing insulin resistance.
